# EUROPEAN PATENT APPLICATION

(11) **EP 4 682 543 A1**
(43) Date of publication of application: **21.01.2026**
(21) Application number: 24770301.0
(22) Date of filing: 06.02.2024
(51) Int. Cl.: G01N 35/00, G06F 3/048

(54) **SAMPLE-PROCESSING SYSTEM AND PROGRAM**

(30) Priority: 13.03.2023 JP 2023038301
(71) Applicant: HITACHI HIGH-TECH CORPORATION, Tokyo 105-6409 (JP)
(72) Inventor: MUGHAL Imama, Tokyo 105-6409 (JP); NITTA Hiroaki, Tokyo 105-6409 (JP); HANAWA Toshihide, Tokyo 105-6409 (JP); WAKUI Akihito, Tokyo 105-6409 (JP)
(74) Representative: Strehl & Partner mbB
(86) International application number: PCT/JP2024/003898
(87) International publication number: WO 2024/190181

(57) **Abstract**

The purpose of the present invention is to provide a sample-processing system and a program with which the need for a screen transition from a main screen to a sub-screen is eliminated in order to avoid interruption to an operation relating to main work. This sample-processing system comprises a sample-processing unit for processing a sample, and a display unit for performing a display relating to the sample. The display unit displays a main screen and an auxiliary screen on which a plurality of user support items are displayed in a switchable manner (see fig. 2).

## Description

### Technical Field

The present invention relates to a sample-processing system and a program.

### Background Art

As a sample-processing system for processing a sample such as blood or urine, there is a system including a transport device that transports a rack accommodating a sample, a system including an automatic analyzer that automatically analyzes a liquid mixture of a sample and a reagent by various measurement methods, or a system combining these systems.

A user who uses an automatic analyzer in the related art performs a screen operation in a state where only a main screen is displayed during main work. In order to display a screen (auxiliary screen), which displays supportive information necessary for the work, on a screen independent of the main screen, it is necessary to temporarily interrupt the operation of the main work.

PTL 1 discloses that "a menu screen is displayed on a display unit of a sample-processing device. When an error occurs in a measurement unit, an error button is displayed, and a help dialog is displayed at an upper portion of an operation portion. The help dialog includes an error message list for displaying detected error items and a detailed procedure display button. When the detailed procedure display button is pressed, a corresponding page of an electronic manual in which an operation procedure for eliminating the error selected in the error message list is described is displayed in a main area. An icon included in the page is linked to a page of the electronic manual that describes an item marked with the icon in more detail".

### Citation List

### Patent Literature

PTL 1: JP2016-053582A

### Summary of Invention

### Technical Problem

When the user operates an analysis unit on the main screen, it may be necessary to check information that is not displayed on the main screen. In such a case, it is necessary for the user to temporarily interrupt the main work, make a screen transition to a sub-screen, check information on the sub-screen, and then make a screen transition to the main screen again. The screen transition requires a specific operation, which takes effort.

In addition, an alarm may be generated due to omission of checking. In PTL 1, the help dialog is displayed at a timing when an error occurs in the measurement unit, and an operation relating to the main work may be interrupted in order to check content of the help dialog.

The invention has been made to solve such a problem, and an object of the invention is to provide a sample-processing system and a program with which the need for a screen transition from a main screen to a sub-screen is eliminated in order to avoid interruption to an operation relating to main work.

### Solution to Problem

An example of a sample-processing system according to the invention is a sample-processing system including:
a sample-processing unit configured to process a sample; and
a display unit configured to perform display related to the sample, in which
the display unit displays a main screen and an auxiliary screen that displays a plurality of user support items in a switchable manner.

An example of a program according to the invention is a program for causing a processor of a sample processing system to execute:
processing a sample; and
performing display related to the sample, in which
the performing the display includes displaying a main screen and an auxiliary screen that displays a plurality of user support items in a switchable manner.

### Advantageous Effects of Invention

According to the invention, a user can check various types of information on the auxiliary screen without screen transition, and thus there is no need for screen transition work from the main screen.

### Brief Description of Drawings

[FIG. 1] FIG. 1 is a schematic diagram including an overall configuration of a sample-processing system according to the embodiment.
[FIG. 2] FIG. 2 illustrates a display example on a display unit, and particularly illustrates a display example for an execution status of periodic maintenance on an auxiliary screen.
[FIG. 3] FIG. 3 illustrates a display example for a ToDo list on the auxiliary screen.
[FIG. 4] FIG. 4 illustrates a display example for a maintenance notification list on the auxiliary screen.
[FIG. 5] FIG. 5 illustrates a display example for maintenance support information on the auxiliary screen.
[FIG. 6] FIG. 6 illustrates a display example for a maintenance video list on the auxiliary screen.
[FIG. 7] FIG. 7 illustrates a display example for a maintenance video on the auxiliary screen.
[FIG. 8] FIG. 8 illustrates a display example for an instruction manual on the auxiliary screen.
[FIG. 9] FIG. 9 illustrates a display example for a shared memo on the auxiliary screen.
[FIG. 10] FIG. 10 illustrates a display example for a menu of a shared memo on the auxiliary screen.
[FIG. 11] FIG. 11 illustrates a display example for a live image on the auxiliary screen.
[FIG. 12] FIG. 12 illustrates a display example for reagent information on the auxiliary screen.
[FIG. 13] FIG. 13 illustrates a display example for a reagent detail on the auxiliary screen.
[FIG. 14] FIG. 14 illustrates another display example for the reagent information on the auxiliary screen.
[FIG. 15] FIG. 15 illustrates a display example for an alarm list on the auxiliary screen.

### Description of Embodiments

Hereinafter, embodiments of the invention will be described with reference to the accompanying drawings, and the invention is not limited to these descriptions.

### [Embodiment 1]

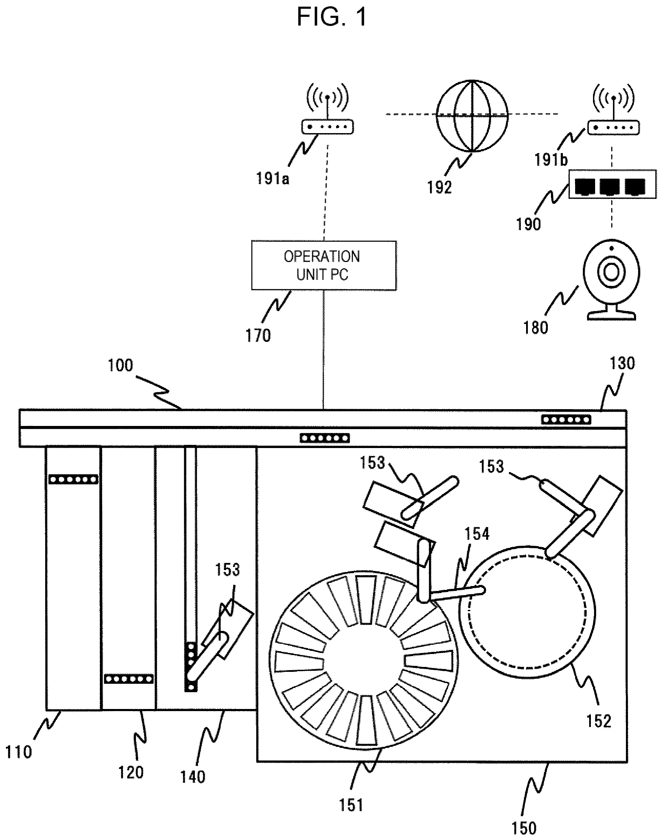

Hereinafter, an embodiment of the invention will be described with reference to FIG. 1.

FIG. 1 is a schematic diagram including an overall configuration of a sample-processing system according to the embodiment. In the embodiment, a sample-processing system is implemented as an automatic analyzer 100. The sample-processing system may include the automatic analyzer 100 and other devices. As described below, the automatic analyzer 100 is a device that analyzes components of a sample by at least one of colorimetric analysis and electrolyte analysis.

In FIG. 1, the automatic analyzer 100 schematically includes a rack supply portion 110, a rack storage portion 120, a sample transport portion 130, an electrolyte analysis unit 140, a colorimetric analysis unit 150, an operation portion PC 170, a network camera 180 via the Internet, and the like.

The operation portion PC 170 is connected to a router 191a, the router 191a is connected to a network 192 (the Internet or the like), the network 192 is connected to a router 191b, the router 191b is connected to a PoE hub 190, and the PoE hub 190 is connected to the network camera 180. The network camera 180 captures an image (a still image or a video, particularly a live image) of a facility or a place (a test room, a reception desk, or the like) related to the automatic analyzer 100 and transmits the video to the operation portion PC 170.

The rack supply portion 110 and the rack storage portion 120 are connected by the sample transport portion 130, and the electrolyte analysis unit 140 and the colorimetric analysis unit 150 are disposed along the sample transport portion 130. The electrolyte analysis unit 140 is an example of a sample-processing unit that processes a sample, and executes electrolyte analysis processing on the sample. The colorimetric analysis unit 150 is also an example of a sample-processing unit that processes a sample, and executes colorimetric analysis processing on the sample.

A sample rack accommodating a sample and a quality control sample is loaded into the rack supply portion 110. The sample rack loaded into the rack supply portion is transported to the electrolyte analysis unit 140, the colorimetric analysis unit 150, and the rack storage portion 120 by the sample transport portion 130. As described above, the automatic analyzer 100 has a function of accommodating and transporting a rack. The rack supply portion 110 and the rack storage portion 120 are examples of a sample-processing unit that processes a sample, and execute storage processing and transport processing on the sample.

Identification information such as a barcode or RFID is attached to the sample rack. The identification information may include information indicating a measurement item set for the sample or the quality control sample.

The electrolyte analysis unit 140 and the colorimetric analysis unit 150 analyze the sample and the quality control sample based on a measurement item requested from the operation portion PC 170. Each analysis unit includes a dispensing probe 153 that dispenses the sample or the quality control sample transported by the sample transport portion 130. The colorimetric analysis unit is provided with a probe 154, and the probe 154 dispenses a reagent into a reaction container of a reaction disk 152 from a reagent disk 151 storing a plurality of reagent bottles containing the reagent used for analysis of the sample.

The operation portion PC 170 controls the units provided in the automatic analyzer 100. The operation portion PC 170 has a hardware structure as a known computer, and includes, for example, a calculation unit and a storage unit. The calculation unit includes, for example, a processor, and the storage unit includes, for example, a storage medium such as a semiconductor memory device and a magnetic disk device. A part or the whole of the storage medium may be a non-transitory storage medium.

The computer may include an input and output unit. The input and output unit includes an input device such as a keyboard and a mouse, an output device such as a display and a printer, and a communication device such as a network interface.

The storage unit may store a program. The computer may execute the functions described in the embodiment by a processor (a processor of the operation portion PC 170 in the embodiment) of the automatic analyzer 100 executing the program.

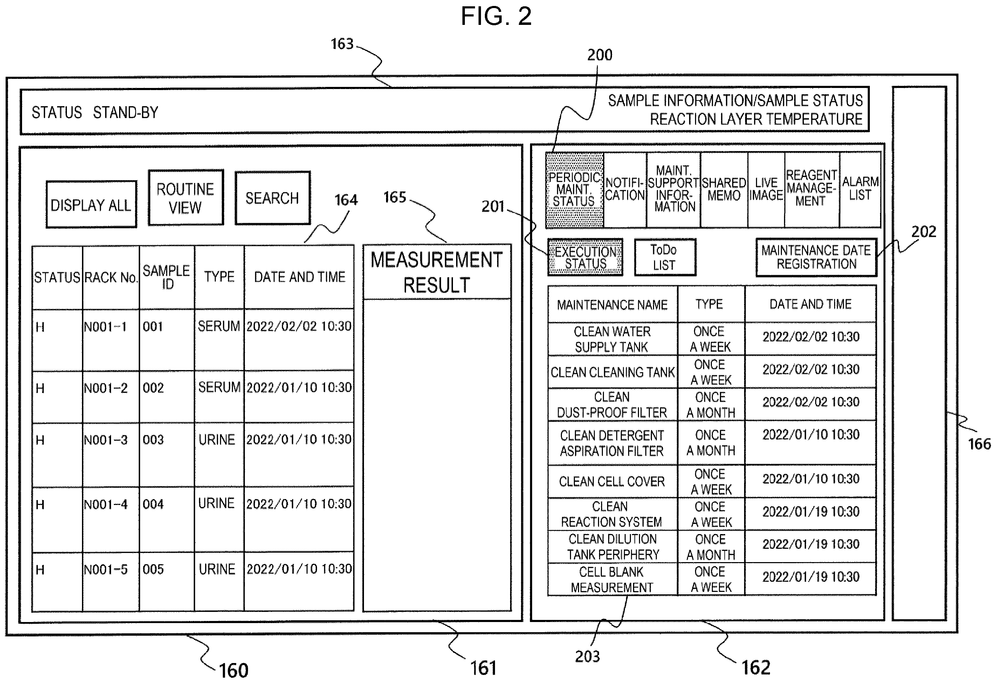

FIG. 2 illustrates a display example on a display unit that performs display related to a sample, and particularly illustrates a display example for an execution status of periodic maintenance on an auxiliary screen. The operation portion PC 170 includes a display unit 160. The display unit 160 displays a main screen 161 and an auxiliary screen 162. The main screen 161 and the auxiliary screen 162 are displayed at the same time in the example in FIG. 2, so that the user's convenience is improved. The main screen 161 and the auxiliary screen 162 may not be displayed simultaneously. The main screen 161 and the auxiliary screen 162 are displayed side by side in the example in FIG. 2, and may not be displayed side by side.

The main screen 161 displays measurement sample information 164, a measurement result 165, a status bar 163 for displaying a sample information status and a device status, and a list 166 of a plurality of types of items related to work occurring during a routine. The main screen 161 may display these in a switchable manner.

The main screen 161 displays information on a sample and test items related to the sample in the measurement sample information 164. Specific content of the information on the sample can be appropriately designed by those skilled in the art. In the example in FIG. 2, the content includes a condition of the sample in the analysis processing, a rack number of a rack in which the sample is accommodated, a sample ID for identifying samples from each other, a date and time related to the sample (for example, a date and time when the sample is collected), and the like. Similarly, the test items related to the sample can be appropriately designed by those skilled in the art. In the example in FIG. 2, items related to serum or items related to urine are designated as the "type". With the main screen 161, the user can appropriately perform main work (for example, work related to a test).

The auxiliary screen 162 is used to check information that is not displayed on the main screen 161. The auxiliary screen 162 displays a plurality of user support items in a switchable manner. Content of the plurality of user support items can be appropriately designed by those skilled in the art, and includes, for example, at least one of a periodic maintenance status, maintenance support information, a shared memo, a maintenance notification, a facility image, reagent information, and an alarm list. By preparing such user support items, the user can check various types of information as described below.

Accordingly, the user can view a screen desired to be checked at any time using the auxiliary screen 162. Further, there is no need to make a screen transition from the main screen 161 in order to check the auxiliary screen 162.

In the example in FIG. 2, the periodic maintenance status is displayed. When a periodic maintenance status button 200 is operated on the auxiliary screen 162, the periodic maintenance status is displayed. The periodic maintenance status includes an execution status and a ToDo list. According to such a configuration, the user can appropriately check the execution status of the periodic maintenance and the ToDo list.

When an execution status button 201 is operated, a maintenance status table 203 is displayed. The maintenance status table 203 associates a maintenance name, a type (execution frequency of maintenance work), and a date and time (date and time when maintenance work was last executed) with each time of work related to the periodic maintenance of the automatic analyzer 100.

The user can register new work of the periodic maintenance by operating a maintenance date registration button 202.

### [Embodiment 2]

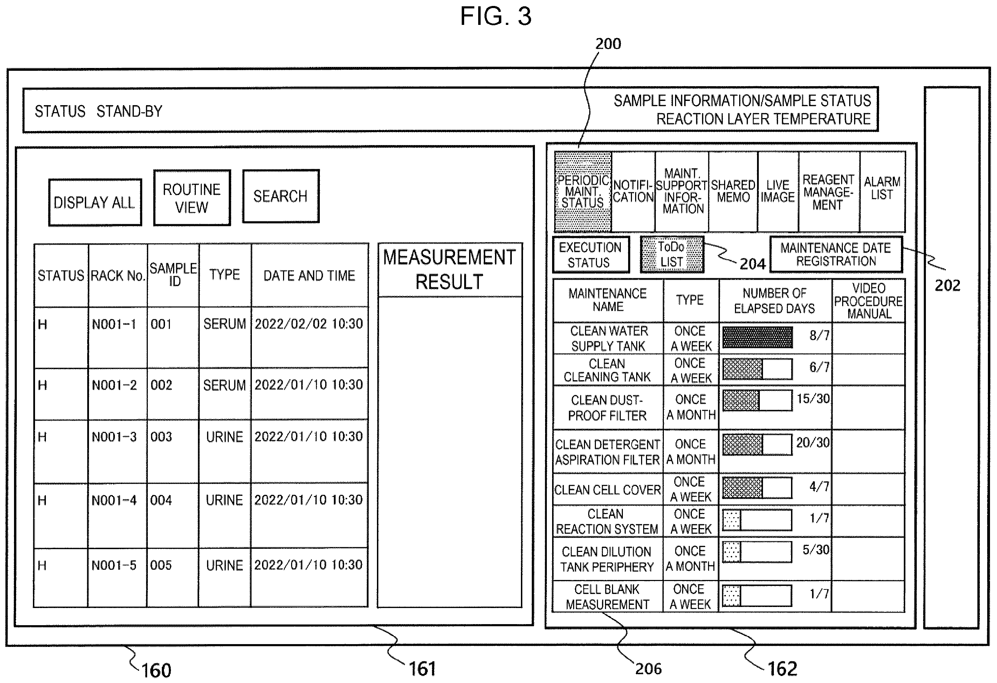

FIG. 3 illustrates a display example for a ToDo list 206 on the auxiliary screen 162. When a ToDo list button 204 is operated, the ToDo list 206 is displayed. The ToDo list 206 associates the maintenance name, the type (execution frequency of the maintenance work), the number of elapsed days (the number of elapsed days from the date and time when the maintenance work was last executed), and the like with each time of work related to the periodic maintenance of the automatic analyzer 100. The execution frequency is, for example, once a month or once a week.

In the ToDo list 206, display with different colors is performed according to a current date and a date on which maintenance is to be executed. The date on which the maintenance is to be executed is a date obtained by adding the execution frequency (for example, one month or one week) to the date on which the maintenance was last executed.

For example, if the current date is past the date on which the maintenance is to be executed, the number of elapsed days is displayed in red (indicated by a dark pattern in FIG. 3). If the current date is the same as or before the date on which the maintenance is to be executed, the number of elapsed days is displayed in another color. For example, when a period from the current date to the date on which the maintenance is to be executed is equal to or shorter than a half cycle, the number of elapsed days is displayed in yellow (indicated by a moderate pattern in FIG. 3). If the period from the current date to the date on which the maintenance is to be executed is equal to or longer than the half cycle, the number of elapsed days is displayed in green (indicated by a light pattern in FIG. 3).

As described, by performing display with different colors, the user can easily understand the degree of urgency of the maintenance work.

The ToDo list 206 may include display (such as a hyperlink) for displaying a video and/or a procedure manual related to the maintenance work.

### [Embodiment 3]

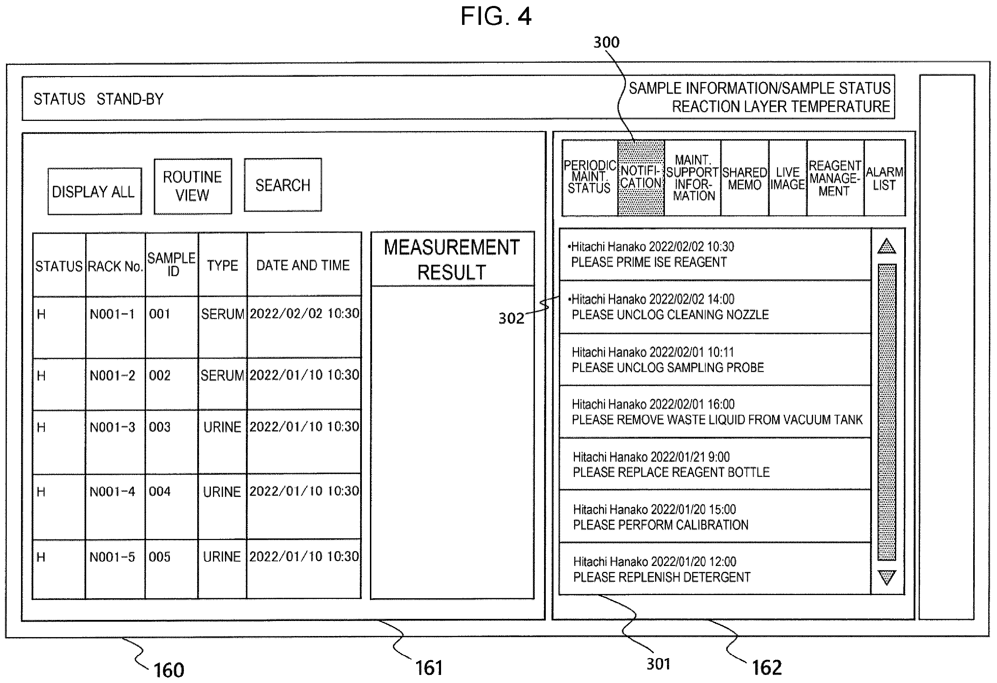

FIG. 4 illustrates a display example for a maintenance notification list 301 on the auxiliary screen 162. When a notification button 300 is operated on the auxiliary screen 162, the maintenance notification list 301 (maintenance notification) is displayed.

The maintenance notification list 301 is a list for notifying the user to execute maintenance to be performed non-periodically. The maintenance notification list 301 includes, for example, at least one of a reagent replacement notification (notification prompting reagent replacement), a calibration notification (notification prompting calibration), a lamp replacement notification (notification prompting lamp replacement), and a detergent replenishment notification (notification prompting detergent replenishment). The maintenance notification includes a message prompting the execution of the maintenance work. In the maintenance notification list 301, an unread mark 302 may be displayed for an unread notification.

With such display, the user can appropriately recognize the necessity of maintenance. When a state in which the maintenance is not executed continues for a long time, an alarm may be generated to stop the analysis operation, but such a situation can be avoided.

### [Embodiment 4]

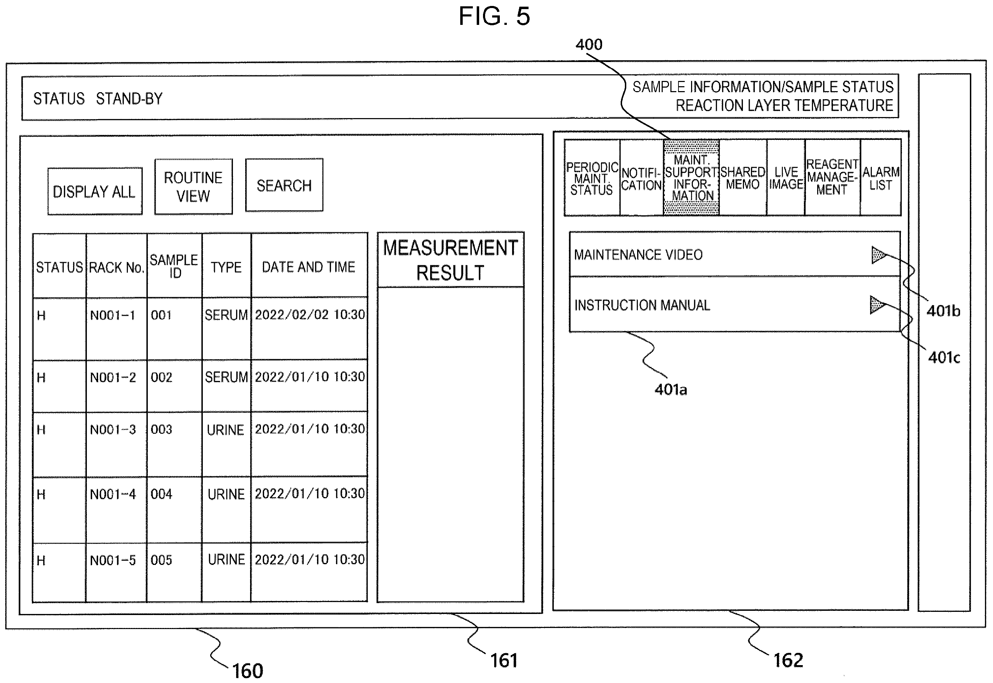

FIG. 5 illustrates a display example for maintenance support information 401a on the auxiliary screen 162. When a maintenance support information button 400 is operated on the auxiliary screen 162, the maintenance support information 401a is displayed.

The maintenance support information 401a includes at least one of an instruction manual and a maintenance video indicating a maintenance procedure. In the example in FIG. 5, a maintenance video button 401b and an instruction manual button 401c are displayed. With such display, the user can appropriately refer to guidance related to the necessary maintenance.

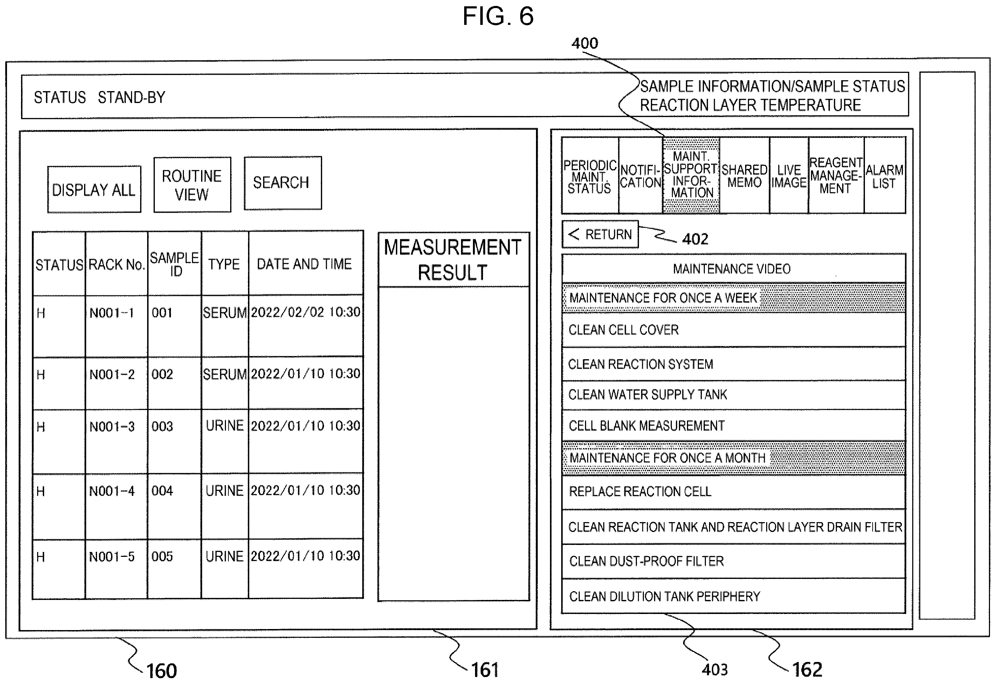

FIG. 6 illustrates a display example for a maintenance video list 403 on the auxiliary screen 162. When the maintenance video button 401b is operated, the maintenance video list 403 in FIG. 6 is displayed. When a return button 402 in FIG. 6 is operated, the screen returns to the screen in FIG. 5.

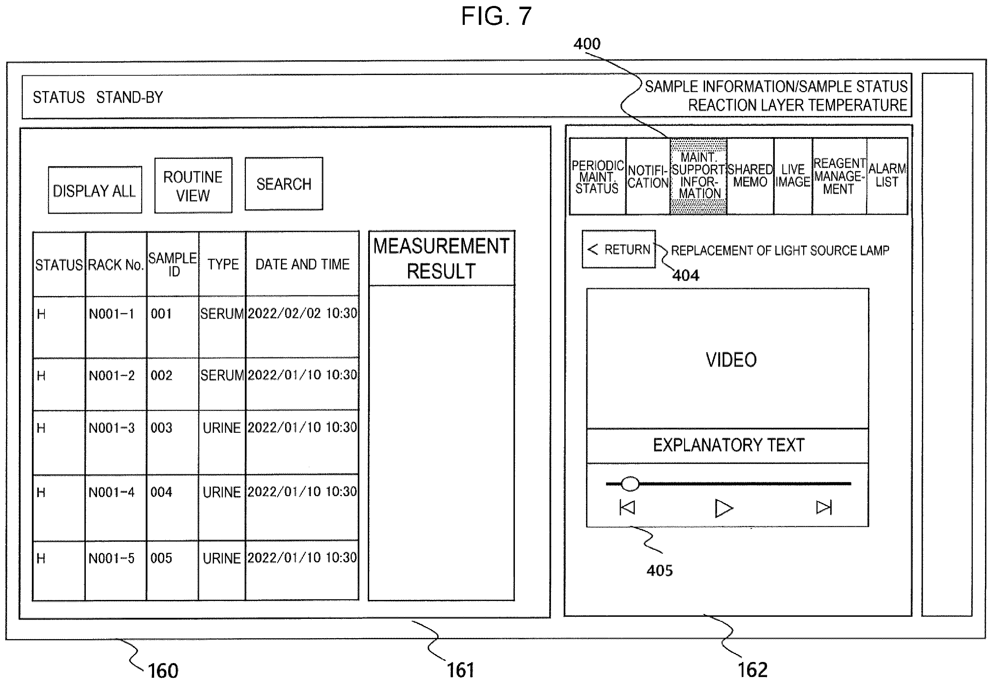

FIG. 7 illustrates a display example for a maintenance video 405 on the auxiliary screen 162. When any video is selected in the maintenance video list 403 in FIG. 6, the maintenance video 405 in FIG. 7 is displayed. The maintenance video 405 includes a video and an explanatory text. With such display, it is possible to execute the maintenance while referring to the video in a state where the main screen 161 is displayed. Accordingly, by referring to not only the description by text but also the video, it is possible to execute the maintenance without mistakes or confusion. When a return button 404 in FIG. 7 is operated, the screen returns to the screen in FIG. 6.

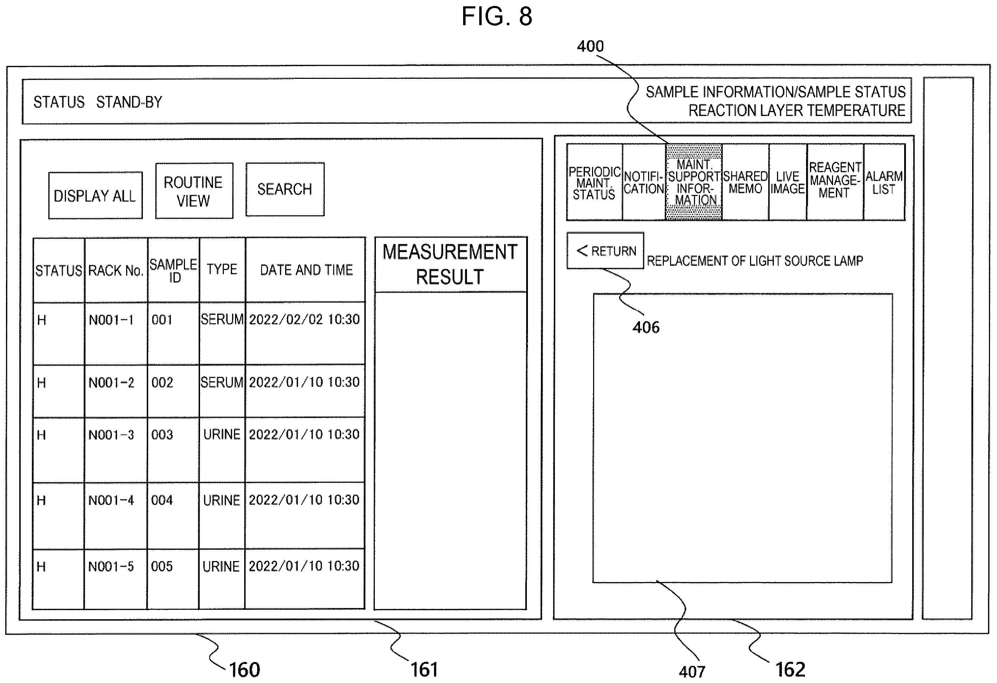

FIG. 8 illustrates a display example for an instruction manual 407 on the auxiliary screen 162. When the instruction manual button 401c is operated in the maintenance support information 401a in FIG. 5, the instruction manual 407 in FIG. 8 is displayed. The instruction manual 407 includes information for explaining a maintenance procedure (for example, at least one of a still image and an explanatory text although not shown in FIG. 8). In this way, content of the instruction manual 407 can be checked on the screen, which leads to a reduction in work time. When a return button 406 in FIG. 8 is operated, the screen returns to the screen in FIG. 5.

### [Embodiment 5]

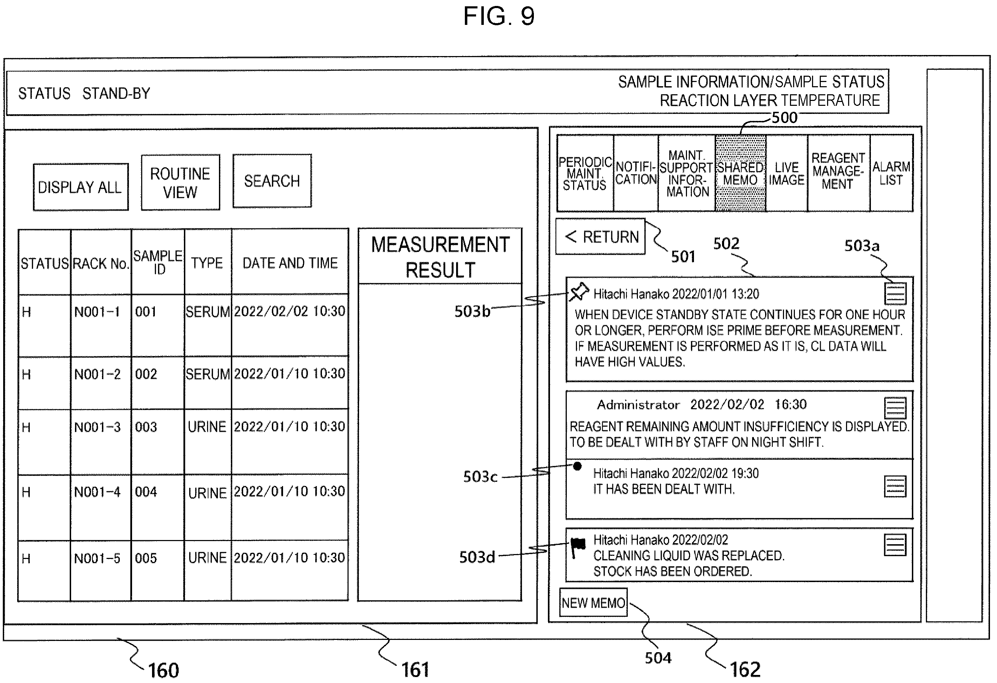

FIG. 9 illustrates a display example for a shared memo 502 on the auxiliary screen 162. The shared memo is an example of a user memo (for example, text data input by the user). When a shared memo button 500 is operated on the auxiliary screen 162, the shared memo 502 is displayed. By displaying the shared memo 502, the user can freely input, record, or transmit a message to the user or another user.

Regarding the shared memo 502, the memo input by the user can be registered, fixed, flagged, edited, replied, and deleted. A new memo button 504 is a button for storing (registering) the new shared memo 502, and when this button is operated, the automatic analyzer 100 receives an input of a new shared memo and registers the new shared memo in the storage unit.

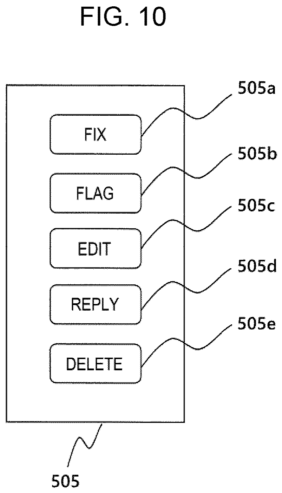

An operation button 503a is displayed for each shared memo 502. When the operation button 503a is operated, a menu 505 shown in FIG. 10 is displayed. FIG. 10 illustrates a display example for the menu 505. The menu 505 includes a fix button 505a, a flag button 505b, an edit button 505c, a reply button 505d, and a delete button 505e.

The fix button 505a is a button for fixing and displaying a shared memo at the top (in the first place). When the fix button 505a is operated, the shared memo is displayed above other shared memos (for example, at an upper position on the screen), and a fix icon 503b is displayed. A plurality of shared memos may be displayed in the first place, and in this case, all the shared memos in the first place are displayed above all the other shared memos, and a display order of the shared memos in the first place can be appropriately determined.

The fix button 505a can be used, for example, to indicate to another user a shared memo that is desired to be noted.

The flag button 505b is a button for fixing and displaying a shared memo in the second place. When the flag button 505b is operated, the shared memo is displayed below the shared memo, for which the fixed button 505a is operated, and above the other shared memos. A plurality of shared memos may be displayed in the second place, and in this case, all the shared memos in the second place are displayed below all the shared memos in the first place and above all the other shared memos, and a display order of the shared memos in the second place can be appropriately determined.

The flag button 505b may be a button for displaying a flag 503d for the shared memo. According to such a configuration, importance of the shared memo can be displayed without changing a display position of the shared memo.

The flag button 505b can be used, for example, to indicate to another user a shared memo that is desired to be noted next to the shared memo for which the fix button is used. For example, an important memo can be checked even if the memo is old.

The edit button 505c is a button for editing content of the shared memo. The reply button 505d is a button for inputting a reply to the shared memo. The delete button 505e is a button for deleting the shared memo.

The shared memo 502 can be effectively used for work transfer and information sharing. An unread mark 503c may be displayed for the unread shared memo 502.

### [Embodiment 6]

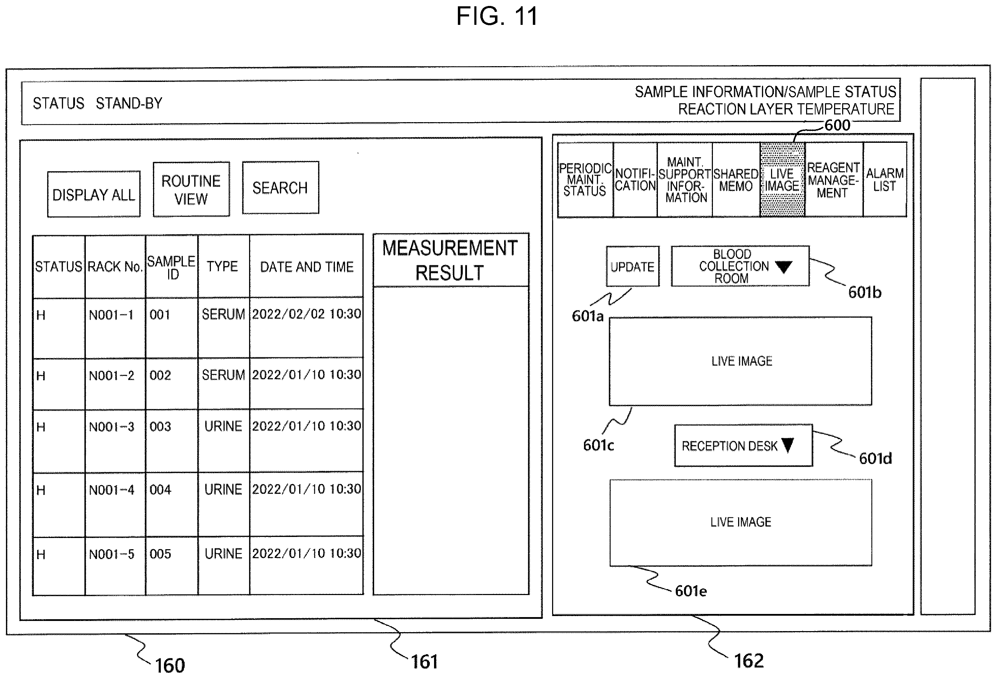

FIG. 11 illustrates a display example for a live image on the auxiliary screen 162. The live image is an example of a facility image. When a live image button 600 is operated on the auxiliary screen 162, a live image 601c and/or a live image 601e are displayed.

For example, the live image 601c is an image of a blood collection room, and the live image 601e is an image of a reception desk. The image is, for example, a video, and may be a still image. As described above, the live image includes at least one of the still image and the video for at least one of the blood collection room and the reception desk. With such display, the user can check a congestion state in each real-time image and grasp a following work capacity. Accordingly, a laboratory technician can smoothly start test work.

The place displayed may be changeable. For example, the user can operate an update button 601a after designating a place by pulldown lists 601b and 601d to display an image of the designated place.

### [Embodiment 7]

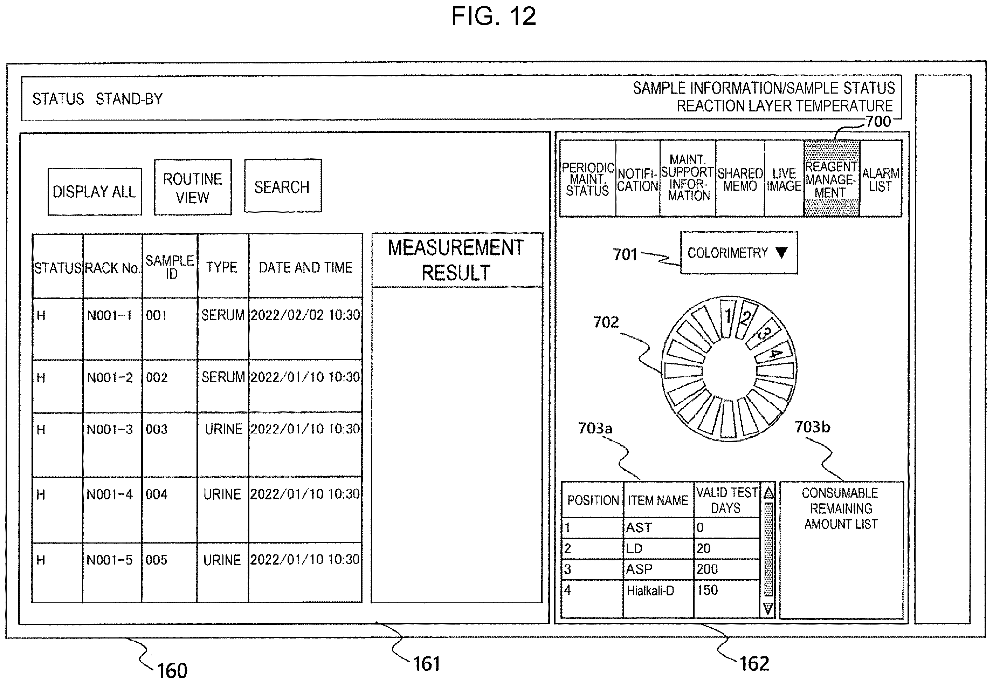

FIG. 12 illustrates a display example for the reagent information on the auxiliary screen 162. When a reagent management button 700 is operated on the auxiliary screen 162, the reagent information is displayed. In the example in FIG. 12, reagent information related to colorimetric analysis is displayed.

FIG. 12 shows reagent information when the colorimetric analysis is selected in an analysis method selection list 701. For a reagent position 702 related to the colorimetric analysis of the automatic analyzer 100, a reagent in use or not in use is displayed in a different color. The reagent information includes reagent usage related to the colorimetric analysis. For example, a reagent remaining amount list 703a for reagents and a consumable remaining amount list 703b are displayed.

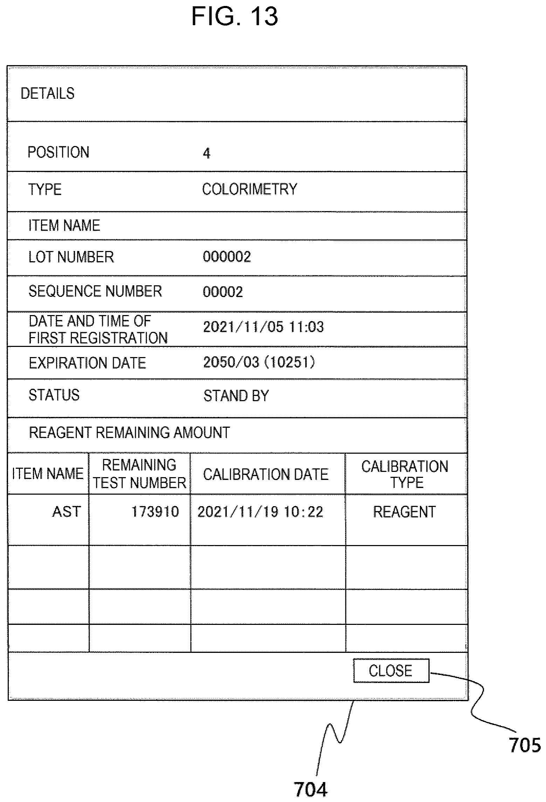

When a specific reagent is selected from the reagent remaining amount list 703a, a reagent detail 704 in FIG. 13 is displayed. The reagent detail 704 include various types of information on the reagent. When a close button 705 in FIG. 13 is operated, the screen in FIG. 13 is closed.

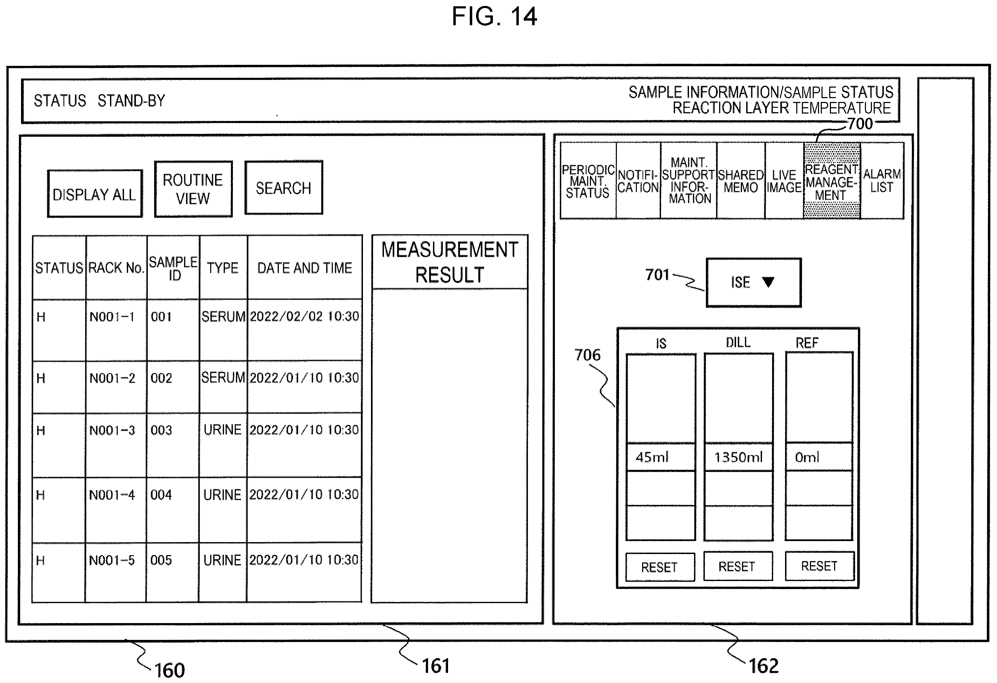

FIG. 14 illustrates another display example for the reagent information on the auxiliary screen 162. In particular, FIG. 14 shows reagent information when an ion selective electrode (ISE) is selected in the analysis method selection list 701. The reagent information includes reagent usage related to the ISE. For example, a remaining amount state 706 of each reagent is displayed.

By displaying such reagent information, it is possible to check in advance that the remaining amount of the reagent is small, register the remaining amount of the reagent in advance, and avoid interruption of the analysis.

### [Embodiment 8]

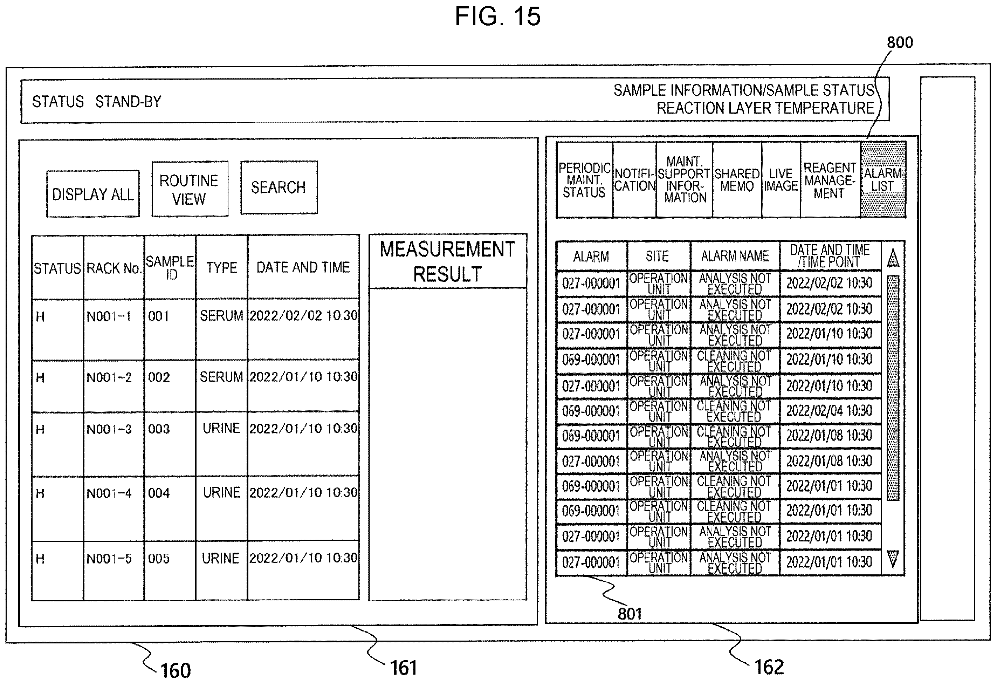

FIG. 15 illustrates a display example for an alarm list 801 on the auxiliary screen 162. When an alarm list button 800 is operated on the auxiliary screen 162, the alarm list 801 is displayed. The alarm list 801 includes all alarms stored in the automatic analyzer 100. The automatic analyzer 100 includes an alarm generated and output by the automatic analyzer 100 itself, an alarm received from the outside of the automatic analyzer 100 and output by the automatic analyzer 100, and the like. When the same type of alarm is generated a plurality of times, the alarms related to the respective times of generation are individually included.

Definition and content of the alarm can be appropriately designed by those skilled in the art, and for example, the following definition is possible. The alarm may be a warning for a state that is not preferable to be left in the automatic analyzer 100. The alarm may include a type of alarm by which the operation of the automatic analyzer 100 is stopped in response to its output, and a type of alarm (warning) by which the operation of the automatic analyzer 100 is not stopped in response to its output. The type of alarm by which the operation of the automatic analyzer 100 is stopped may include a type of alarm by which only a sample sampling operation is stopped after the alarm is generated, a type of alarm by which the entire automatic analyzer 100 is to be stopped at the time when a currently executed operation is completed after the alarm is generated, and a type of alarm by which the entire automatic analyzer 100 is stopped immediately at the time when the alarm is generated.

When the automatic analyzer 100 detects a predetermined condition defined as an alarm trigger, the automatic analyzer 100 may output an alarm in response to the detection and display a countermeasure method to the user.

In a configuration in the related art, even if the same alarm is output twice or more, since only one alarm is output on the screen, the user needs to print accumulated information of the alarms and check all the past alarms every time in order to check the device status, which takes time and effort. On the other hand, according to the embodiment, when an alarm is displayed on the main screen 161, all alarms including the alarm can be checked on the auxiliary screen 162, and thus it takes no time and effort for checking.

As described above, according to the embodiments of the invention, since the user can check various types of information on the auxiliary screen, the screen transition work from the main screen is unnecessary.

### [Other Embodiments]

The sample-processing system is not limited to one having an analysis function like the automatic analyzer 100. For example, a sample-processing system having only a function of storing and transporting a rack accommodating samples can be also implemented.

### Reference Signs List

100: automatic analyzer (sample-processing system)
110: rack supply portion
120: rack storage portion
130: sample transport portion
140: electrolyte analysis unit
150: colorimetric analysis unit
151: reagent disk
152: reaction disk
153: dispensing probe
154: probe
160: display unit
161: main screen
162: auxiliary screen
163: status bar
164: measurement sample information
165: measurement result
166: list of plurality of types of items related to work
170: operation portion PC
180: network camera
190: PoE hub
191a, 191b: router
192: network
200: periodic maintenance status button
201: execution status button
202: maintenance date registration button
203: maintenance status table
204: ToDo list button
206: ToDo list
300: notification button
301: maintenance notification list
302: unread mark
400: maintenance support information button
401a: maintenance support information
401b: maintenance video button
401c: instruction manual button
402: return button
403: maintenance video list
404: return button
405: maintenance video
406: return button
407: instruction manual
500: shared memo button
502: shared memo (user memo)
503a: operation button
503b: fix icon
503c: unread mark
504: new memo button
505: menu
505a: fix button
505b: flag button
505c: edit button
505d: reply button
505e: delete button
600: live image button
601a: update button
601b: pulldown list
601c: live image
601d: pulldown list
601e: live image
700: reagent management button
701: analysis method selection list
702: reagent position
703a: reagent remaining amount list
703b: consumable remaining amount list
704: reagent detail
705: close button
706: reagent remaining amount state
800: alarm list button
801: alarm list

## Claims

1. A sample-processing system comprising:
a sample-processing unit configured to process a sample; and
a display unit configured to perform display related to the sample, wherein
the display unit displays a main screen and an auxiliary screen that displays a plurality of user support items in a switchable manner.

2. The sample-processing system according to claim 1, wherein
the plurality of user support items include at least one of a periodic maintenance status, maintenance support information, a user memo, a maintenance notification, a facility image, reagent information, and an alarm list.

3. The sample-processing system according to claim 2, wherein
the plurality of user support items include the periodic maintenance status, and
the periodic maintenance status includes an execution status and a ToDo list.

4. The sample-processing system according to claim 3, wherein
in the ToDo list, display with different colors is performed according to a current date and a date on which maintenance is to be executed.

5. The sample-processing system according to claim 2, wherein
the plurality of user support items include the maintenance support information, and
the maintenance support information includes at least one of an instruction manual and a maintenance video indicating a maintenance procedure.

6. The sample-processing system according to claim 5, wherein
the maintenance video includes a video and an explanatory text, and
the instruction manual includes at least one of a still image and an explanatory text.

7. The sample-processing system according to claim 2, wherein
the plurality of user support items include the user memo.

8. The sample-processing system according to claim 7, wherein
the display unit displays a new memo button for storing a new user memo, and
the display unit displays, for one designated user memo,
a fix button for displaying the user memo in a first place,
a flag button for displaying the user memo in a second place,
an edit button for editing content of the user memo,
a reply button for inputting a reply to the user memo, and
a delete button for deleting the user memo.

9. The sample-processing system according to claim 2, wherein
the plurality of user support items include the maintenance notification, and
the maintenance notification includes at least one of a reagent replacement notification, a calibration notification, a lamp replacement notification, and a detergent replenishment notification.

10. The sample-processing system according to claim 2, wherein
the plurality of user support items include the facility image, and
the facility image includes at least one of a still image and a video for at least one of a blood collection room and a reception desk.

11. The sample-processing system according to claim 2, wherein
the plurality of user support items include the reagent information, and
the reagent information includes reagent usage related to colorimetric analysis and reagent usage related to an ion selective electrode.

12. The sample-processing system according to claim 2, wherein
the plurality of user support items include the alarm list, and
the alarm list includes all alarms stored in the sample-processing system.

13. The sample-processing system according to claim 1, further comprising:
an automatic analyzer configured to perform at least one of colorimetric analysis and electrolyte analysis.

14. The sample-processing system according to claim 1, wherein
the main screen displays information on the sample and test items related to the sample.

15. The sample-processing system according to claim 1, wherein
the display unit simultaneously displays the main screen and the auxiliary screen.

16. A program for causing a processor of a sample- processing system to execute:
processing a sample; and
performing display related to the sample, wherein
the performing the display includes displaying a main screen and an auxiliary screen that displays a plurality of user support items in a switchable manner.
